(19) Europäisches Patentamt
European Patent Office
Office européen des brevets

(11) Publication number: 0 001 651
B1

(12) EUROPEAN PATENT SPECIFICATION

(45) Date of publication of patent specification: 12.05.82

(51) Int. Cl.[3]: C 07 C 41/06, C 07 C 43/10, C 07 C 43/11

(21) Application number: 78200207.5

(22) Date of filing: 25.09.78

# (54) A process for preparing monoalkyl ethers of alkanediols or oxa-alkanediols.

(30) Priority: 03.10.77 US 838506

(43) Date of publication of application: 02.05.79 Bulletin 79/9

(45) Publication of the grant of the patent: 12.05.82 Bulletin 82/19

(84) Designated Contracting States: DE FR GB NL

(56) References cited:
US - A - 3 431 308

Chemical Abstracts vol 76, no. 9 1972
Columbus, Ohio, USA
N. TOKURA "Recent developments in organic synthesis in liquid sulfur dioxide"
page 309, column 1, abstract no. 45254w

Chemical Abstract vol 64, no. 3, 1966
Columbus, Ohio, USA
"Alkyl hydroxyalkyl ether from olefins and polyols"
column 3354 g-h

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Kim, Leo
12126 Westmere Drive
Houston Texas 77077 (US)
Inventor: Paxson, Timm Edward
15910 Haven Hills Drive
Houston Texas 77084 (US)

(74) Representative: Puister, Antonius Tonnis, Mr. et al,
P.O. Box 302
NL-2501 CH The Hague (NL)

EP 0 001 651 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# A process for preparing monoalkyl ethers of alkanediols or oxa-alkanediols

The invention is concerned with a process for preparing monoalkyl ethers of alkanediols or oxa-alkanediols by reacting one or more olefins with one or more alkanediols or oxa-alkanediols.

Monoalkyl ethers of alkanediols or oxa-alkanediols prepared by reacting an olefin with an alkanediol or oxa-alkanediol may be useful as secondary alcohol alkoxylate detergents or as intermediates in the preparation thereof. Secondary alcohol ethoxylates generally have low pour points and good wetting and solubility properties which make them particularly suitable in industrial detergent applications.

It is known to prepare monoalkyl ethers of alkanediols or oxa-alkanediols by reacting an olefin with an alkanediol or oxa-alkanediol at a temperature of from 20°C to 250°C in the presence of an acid catalyst and a polar solvent. Examples of suitable acids include $BF_3$, sulphuric acids and aromatic sulphonic acids and examples of suitable polar solvents include nitromethane and sulfolane, e.g. see Netherlands Patent Specification No. 111,296. Another example of a suitable polar solvent is a mixture of on the one hand sulfolane and/or a methyl derivative thereof and on the other hand a nitro compound having not more than eight carbon atoms and/or an aliphatic halogen compound having not more than four carbon atoms, see Netherlands Patent Application No. 6401246.

It has now been found that if liquid sulphur dioxide is used as the polar solvent an improved conversion of olefin and enhanced selectivities to the monoalkyl ether of the alkanediol or oxa-alkanediol are obtained. One advantage of the process of the invention is that the particular alkanediol or oxa-alkanediol reactant selectively adds cleanly to the reactant olefin without polymerizing to higher alkanediols or oxa-alkanediols.

Accordingly, the present invention is concerned with a process for preparing monoalkyl ethers of alkanediols or oxa-alkanediols comprising reacting one or more olefins with one or more alkanediols or oxa-alkanediols at a temperature of from 20°C to 250°C in the presence of an acid catalyst and a polar solvent, characterized in that the polar solvent is sulphur dioxide.

Suitable olefins for use in the present invention are linear or branched olefins having from 2 to about 40 carbon atoms. The process of this invention is particularly suited to detergent range olefins, i.e., those containing from 6, suitably 8 to 20 carbon atoms per molecule. Prior art processes react detergent range olefins with difficulty, particularly detergent range internal olefins. The words "internal olefins" refer to olefins which do not have terminal carbon atoms forming a carbon-carbon double bond. Mixtures of olefins may be utilized.

Suitable alkanediols or oxa-alkanediols for use in the present invention are dihydric alcohols having from 2 to about 30 carbon atoms per molecule. They include dihydric alkanols, such as ethylene glycol, propylene glycol, butylene glycol, their homologues and the like, as well as the polymeric derivatives of dihydric alkanols, such as diethylene glycol, triethylene glycol, tetraethylene glycol, dipropylene glycol, tripropylene glycol, dibutylene glycol, propylene-ethylene glycol, isobutylene-ethylene glycol and the like. Preferred reactants are the (poly)ethylene and (poly)propylene glycols.

The acid catalysts for use in the present invention may be heterogeneous or homogeneous catalysts and may be either Bronsted or Lewis acids. The Bronsted acids preferably have a pKa of not more than about 2.5. Examples are sulphuric acid, fuming sulphuric acid, fluorosulphonic acid; aromatic sulphonic acids such as benzene sulphonic acid, toluene sulphonic acid and related homologues and analogues; alkyl sulphonic acids including the fluorinated sulphonic acids, such as methyl sulphonic acid, perfluoromethylsulphonic acid, perfluoroethyl sulphonic acid, and the like. Heterogeneous catalysts include the acidic ion-exchange resins, such as the sulphonated ion-exchange resins, i.e., those containing a plurality of sulphonic acid groups. Examples of such resins include sulphonated styrene-divinylbenzene copolymers, sulphonated phenolformaldehyde resins and sulphonated benzene-formaldehyde resins. The resin may be of the gel or macroreticular type. Also suitable are the fluoroalkyl sulphonic acid resins, especially the fluoroalkyl sulphonic acid resins having the group

|
$CFSO_3H$
|

as an acidic moiety of the resin, and a pKa of not more than about 2.5. The preferred fluorinated alkyl sulphonic acid resins are those having the formula

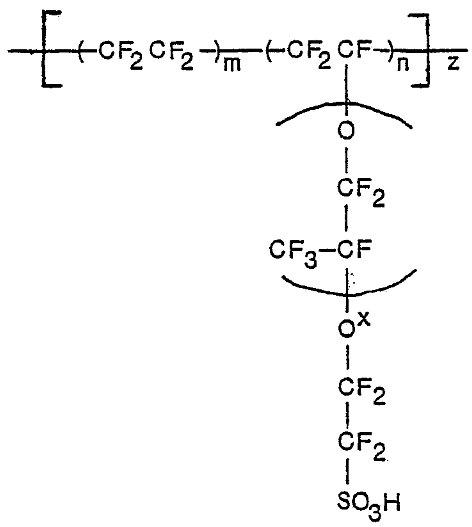

1)

wherein n is at least one and the sum of m, n, x and z are such that the equivalent weight is 2000 or less, preferably between about 900 and about 2000, and most preferably between about 960 and about 1200. These resins are further described in U.S. Patent Specification No. 3,282,875.

The fluoroalkyl sulphonic acid resins can also have the formulae:—

2) $-(CR_2CR_2)_y-$ , 3) 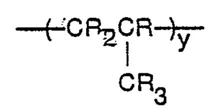

and 4) 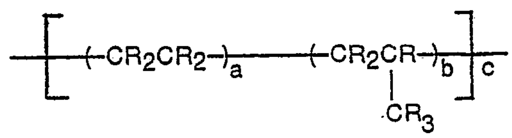

where R is individually a hydrogen, a fluorine and a —$SO_3H$ group and where at least some of the carbons attached to greater than one R simultaneously have one R a —$SO_3H$ and one a fluorine and where y and the sum of a, b and c and R are such that the equivalent weight is less than about 2000, preferably between about 300 and about 1500. A preferred resin is Nafion XR resin supplied by E.I. DuPont de Nemours Co., Inc. (Nafion is a registered Trade Mark). These resins may be used neat, or supported on a carrier.

The preferred Lewis acids are those having heats of formation with pyridine more exothermic than 7 kcal./mol., preferably more exothermic than 9 kcal./mol. Methods of calculation of heats of formation are given in Drago, et al., J. American Chem. Soc., *93* 6014, 1971. Suitable examples are $BF_3$, $SbCl_5$, $AlCl_3$, $AlF_3$, $B(CH_3)_3$ and the like.

The reaction is suitably carried out at a temperature in the range of from 20°C to 250°C, preferably of from 70°C to 200°C and more preferably of from 100°C to 160°C.

The process of the invention may also be carried out in the presence of a polar organic co-solvent. Suitable co-solvents generally exhibit a dielectric constant of at least 5 at the reaction temperature. Suitable examples are nitromethane, nitroethane, nitrobenzene, dioxane, 1,2-dimethyloxyethane, furan and sulfolane and its homologues and analogues.

In the process according to this invention, the olefin, alkanediol or oxa-alkanediol, catalyst, sulphur dioxide and optionally co-solvents are suitably brought into contact with each other in a reactor, heated to the reaction temperature at a given sulphur dioxide partial pressure and allowed to react for from about 0.1 to about 30 hours. After cooling, the reactants are worked up by conventional methods to obtain the product monoalkyl ethers or alkanediols or oxa-alkanediols. The use of heterogeneous catalysts allows for the use of flow reactors with liquid hour space velocities ranging from about 0.01 to about 10 $hours^{-1}$. Suitably, the reaction pressure is not higher than 20 MPa. The molar ratio of feed reactants of olefin:(alkanediol or oxa-alkanediol):acid catalyst (hydrogen equivalent):sulphur dioxide is suitably 1:1 to 30:0.01 to 10:1 to 100, and preferably 1:3 to 10:0.1 to 4:5 to 30.

The process of the invention will now be illustrated by reference to the following Examples. In the Examples the reactions were run in either a 300-ml or a 1-litre Hastelloy-B autoclave with or without a glass liner. A Teflon (registered Trade Mark) stirring paddle assembly was employed in conjunction with a Teflon coated or wrapped stirring shaft. The cooling coils and thermocouple well were similarly protected.

The reactor was charged in the following manner: glycol, acid, and finally olefin. The sequence of addition may be important to prevent charring of the olefin. The autoclave was sealed, pressure-tested with $N_2$, and cooled to −20°C before admitting the $SO_2$. The reactor was pressurized to from 3.54 to 10.4 MPa of $N_2$. When a solvent other than $SO_2$ was utilized, such solvent was added together with the olefin.

EXAMPLE 1

A 300-ml, Hastelloy B autoclave with glass liner was charged with 20 g (124 mmol.) of an internal $C_{11}$- and $C_{12}$-olefin mixture, 46.2 g (744 mmol.) of ethylene glycol, 139 g (2170 mmol.) of sulphur dioxide, and 17.5 g (99 mmol.) of benzene sulphonic acid monohydrate in the manner described above. The reaction was heated at 130°C for 2 hours. Work-up and gas chromatographic analysis of the reaction mixture gave the distribution: 17.4:18.9 m% conversion of internal olefin, 6.4 m% selectivity to secondary alcohols, 92.4 m% selectivity to the ethylene glycol-olefin adduct, and 1.6 m% selectivity to the diethylene glycol-olefin adduct.

EXAMPLE 2

The experiment of Example 1 was repeated using equivalent molar amounts with a mixed $C_{13}$- and $C_{14}$-internal olefin feed. Analysis of the product showed a conversion of 16—17.1 m%, 6.5 m% selectivity to secondary alcohols, 91.3 m% selectivity to ethylene glycol-olefin adduct, 1.8 m% selectivity to diethylene glycol-olefin adduct.

EXAMPLE 3

The experiment of Example 1 was repeated using a $H_2SO_4/SO_3/H_3BO_3$ catalyst, internal $C_{11}/C_{12}$-olefin feedstock, and a mixed sulfolane/$SO_2$ solvent system. The solvent was calculated on the basis of three times the weight of ethylene glycol. A conversion of 6.1—7.0 m% was obtained with a 7 m% selectivity to secondary alcohols, 86 m% selectivity to ethylene glycol-olefin adduct, and 7 m% selectivity to diethylene glycol-olefin adduct.

EXAMPLE 4

Repeating the Experiment of Example 1 with the same molar amounts and using $SO_2$ solvent, but varying the acid catalysts gave the following results:

TABLE 1

| Catalyst | Conversion (m%) | Selectivities (m%) Alcohol | ME[1] | DE[2] |
|---|---|---|---|---|
| $H_2SO_4$ | 4—6 | 7.3—12 | 88—82 | 4.4—6.0 |
| $H_2SO_4/SO_3$ | 5—8 | 7.3—12 | 88—82 | 4.0—6.0 |
| $H_2SO_4/SO_3/H_3BO_3$ | 13.4—14.3 | 7.4 | 88.2 | 4.4 |
| $FSO_3H/SbF_5$ | 17—18 | 4.0 | 90.3 | 5.8 |
| $FSO_3H$ | 8.8 | 8.2 | 84.1 | 5.1 |
| $p\text{-}CH_3C_6H_4SO_3H.H_2O$ | 16.5—19.5 | 6.0 | 92.0 | 2.0 |
| $CH_3SO_3H$ | 10.9—11.9 | 6.6 | 91.0 | 2.4 |
| $m\text{-}HO_3SC_6H_4SO_3H$ | 10.9—11.3 | 13.2—15.3 | 80—77 | 7.9—7.7 |
| $BF_3.H_2O$ | 5.8 | 10 | 86 | 4 |
| $HBF_4$ | 10.9 | 15.4 | 84.6 | — |

1. ME = ethylene glycol-olefin adduct
2. DE = diethylene glycol-olefin adduct

EXAMPLE 5 (comparative)

Example 1 was repeated using different solvents. Molar ratios of olefine: $p\text{-}CH_3C_6H_4SO_3H.H_2O$: ethylene glycol remained at 1:0.8:6. Solvent concentrations were held at 3 times the weight of ethylene glycol. Some solvents were also run with a sulphuric acid catalyst. Time of reaction was 2

hours, temperature 130°C. If the solvent had a boiling point below 130°C, an inert gas cap ($N_2$) was maintained over the reaction.

TABLE 2

| Catalyst | Solvent | Conversion (m%) | Selectivities (m%) Alcohol | ME[1] | DE[2] |
|---|---|---|---|---|---|
| $pCH_3C_6H_4SO_3H.H_2O$ | $CH_2Cl_2$ | 1—2 | 8—10 | 90—88 | 2—4 |
| $pCH_3C_6H_4SO_3H.H_2O$ | 1,2-$Cl_2C_2H_4$ | 3.1 | 8—10 | 90—88 | 2—4 |
| $pCH_3C_6H_4SO_3H.H_2O$ | $(CF_3)_2CHOH$ | 9.6 | ~8 Some $(CF_3)_2CHO$— adduct | ~88 | ~2 |
| $pCH_3C_6H_4SO_3H.H_2O$ | $(CH_3)_2SO$ | 0 | — | — | — |
| $pCH_3C_6H_4SO_3H.H_2O$ | $CH_3CN$ | 0 | — | — | — |
| $pCH_3C_6H_4SO_3H.H_2O$ | $CH_3NO_2$ | 4.2 | 8—10 | 90—88 | 2—4 |
| $H_2SO_4.H_2O$ | $C_4H_8SO_2$ (sulfolane) | 5.0 | 10—12 | 86—84 | 4—6 |
| $H_2SO_4.H_2O$ | 3-methyl (sulfolane) | 4.0—5.0 | 10—12 | 86—84 | 4.6 |
| $H_2SO_4.H_2O$ | $(CH_3)_2SO_2$ | 3.0—3.8 | 10—12 | 86—84 | 4—6 |
| $H_2SO_4.H_2O$ | $C_6H_5NO_2/C_4H_8SO_2$ | 8—10 | 10—12 | 86—84 | 4—6 |

1. ME = ethylene glycol — olefin adduct
2. DE = diethylene glycol — olefin adduct

## EXAMPLE 6

Example 1 was repeated using a sulphonated styrene-divinylbenzene acid ion exchange resin catalyst Dowex — MSC — 1—H in $SO_2$ solvent (Molar ratio olefin:acid ratio 1:0.4). Conversion was 5.3m% after a reaction time of 3 hours at 130°C, 6.8m% selectivity to secondary alcohols, 90.8m% selectivity to ethylene glycol-olefin adduct, and 2.4m% selectivity to the diethylene glycol-olefin adduct. The above reaction was repeated using various sulphonated acid ion exchange resins. Olefin:resin ratios (based on milliequivalents of acid sites) were held at 1:0.5 to 1. Reaction conditions were held at 130°C for 3 hours.

TABLE 3

| Resin | Manufacturer | Conversion (m%) | Selectivities (m%) | | |
|---|---|---|---|---|---|
| | | | Alcohol | ME[1] | DE[2] |
| XN1010 | Rohm and Haas | ~1.0 | not calculated | | |
| XN1011 | Rohm and Haas | ~1.2 | not calculated | | |
| XN1005 | Rohm and Haas | 6.0 | 23.5 | 76.5 | trace |
| XN1008 | Rohm and Haas | 1.2—1.3 | 25.6 | 74.4 | — |
| Amberlyst[3] 15 | Rohm and Haas | 2.8 | 6.0 | 92.0 | 2.0 |
| Amberlyst[3] 252 | Rohm and Haas | 2.2 | 11.0 | 85.0 | 4.0 |
| Dowex[3] MSC—1—H | Dow | 5.3 | 6.8 | 90.8 | 2.4 |
| Duolite[3] ARC—351 | Diamond Shamrock | 1.2 | 14.8 | 74.7 | 10.5 |
| Zeo-Carb[3] 225 SRC—14 | BDH Chemicals | <1.0 | — | — | — |
| AG—50W—X2 | Bio-Rad | 8.1—10.1 | 9 | 88 | 3.0 |

1. ME = ethylene glycol — olefin adduct
2. DE = diethylene glycol — olefin adduct
3. registered Trade Mark

## EXAMPLE 7

A 1-litre autoclave with glass liner was charged with 60 g (.713 moles) of mixed internal hexenes, 232 g (3.74 moles) of ethylene glycol, 25 g of an $H_2SO_4/SO_3/H_3BO_3$ acid mix (molar ratio 1:0.3:0.1), and 300 g (4.68 moles) of sulphur dioxide. The reactor was pressurized to 10.4 MPa of nitrogen. The mixture was heated for 2 hours at 130°C. Upon isolation there was obtained a 44.5m% conversion with a 5.5m% selectivity to secondary hexanols, 84.0m% selectivity to the ethylene glycol-olefin adduct, 2.9m% selectivity to the diethylene glycol-olefin adduct, and 8.0m% selectivity to the ether $C_6H_{13}OCH_2CH_2OC_6H_{13}$.

## EXAMPLE 8

A series of experiments were performed using 1-dodecene and ethylene glycol as reactants, $H_2SO_4$ as catalyst and various solvents. The reactor was pressurized to 10.4 MPa of nitrogen. These results are given in Table 4.

TABLE 4

| Experiment | Catalyst | Solvent | t, °C | Reaction time, min. | Conversion 1-dodecene, %m | Selectivity[1] %m |
|---|---|---|---|---|---|---|
| 1[2,4] | $H_2SO_4$ | Sulfolane | 150 | 120 | 12 | 75% I<br>10% II |
| 2 [3] | $H_2SO_4$ | $SO_2$ | 150 | 50 | 35 | >95% I |
| 3[2,4] | $H_2SO_4$ | $CH_3CN$ | 150 | 60 | 3 | 90% I |
| 4[3] | None | $SO_2$ | 150 | 60 | 0 | — |

1. $C_{10}H_{21}CHCH_3$ = I
   |
   $OCH_2CH_2OH$

   $C_{10}H_{21}CHCH_3$ = II
   |
   $OCH_2CH_2OCH_2CH_2OH$

2. 1.5 g catalyst, 4.0 ml solvent, 1.0 ml 1-dodecene, 2.0 ml ethylene glycol;
3. 60 ml $SO_2$, 10 ml 1-dodecene, 20 ml ethylene glycol, 1 ml $H_2SO_4$ (when present);
4. comparative.

## Claims

1. A process for preparing monoalkyl ethers of alkanediols or oxa-alkanediols comprising reacting one or more olefins with one or more alkanediols or oxa-alkanediols at a temperature of from 20°C to 250°C in the presence of an acid catalyst and a polar solvent, characterized in that the polar solvent is sulphur dioxide.

2. A process as claimed in claim 1, characterised in that the reaction pressure is not higher than 20 MPa.

3. A process as claimed in claim 1 or claim 2, characterized in that the reaction temperature is in the range of from 100°C to 160°C.

4. A process as claimed in any one of claims 1 to 3, characterized in that the polar solvent is a mixture of sulphur dioxide and a polar organic co-solvent.

5. A process as claimed in any one of claims 1—4, characterized in that the olefin is a $C_6$ to $C_{20}$ olefin.

6. A process as claimed in any one of claims 1 to 5, characterized in that the molar ratio of olefin:(alkanediol or oxa-alkanediol):acid catalyst (hydrogen equivalent):sulphur dioxide is 1:1 to 30:0.01 to 10:1 to 100.

7. A process as claimed in any one of claims 1 to 6, characterized in that the acid catalyst is a Bronsted acid having a pKa of not more than 2.5.

8. A process as claimed in any one of claims 1 to 6, characterized in that the acid catalyst is a Lewis acid having a heat of formation with pyridine more exothermic than 9 kcal/mole.

## Revendications

1. Procédé de préparation de monoalkyléthers d'alcanediols ou d'oxa-alcanediols, selon lequel on fait réagir une ou plusieurs oléfines avec un ou plusieurs alcanediols ou oxa-alcanediols à une température comprise entre 20°C et 250°C en présence d'un catalyseur acide et d'un solvant polaire, caractérisé en ce que le solvant polaire est l'anhydride sulfureux.

2. Procédé selon la revendication 1, caractérisé en ce que la pression de réaction n'est pas supérieure à 20 MPa.

3. Procédé selon l'une des revendications 1 et 2, caractérisé en ce que la température de réaction est comprise entre 100°C et 160°C.

4. Procédé selon l'une quelconque des revendications 1 à 3, caractérisé en ce que le solvant polaire est un mélange d'anhydride sulfureux et d'un cosolvant organique polaire.

5. Procédé selon l'une quelconque des revendications 1 à 4, caractérisé en ce que l'oléfine est une oléfine de $C_6$ à $C_{20}$.

6. Procédé selon l'une quelconque des revendications 1 à 5, caractérisé en ce que les rapports molaires oléfine:(alcanediol ou oxa-alcanediol):catalyseur acide (équivalent hydrogène):anhydride sulfureux sont de 1:1 à 30:0,01 à 10:1 à 100.

7. Procédé selon l'une quelconque des revendications 1 à 6, caractérisé en ce que le catalyseur acide est un acide de Bronsted ayant un pKa de pas plus de 2,5.

8. Procédé selon l'une quelconque des revendications 1 à 6, caractérisé en ce que le catalyseur acide est un acide de Lewis ayant une chaleur de formation avec la pyridine plus exothermique que 9 kilocalories par mole.

## Patentansprüche

1. Ein Verfahren zur Hestellung von Monoalkyläthern von Alkandiolen oder Oxa-alkandiolen, die Massnahme umfassend, dass ein oder mehrere Olefine mit einem oder mehreren Alkandiolen oder Oxa-alkandiolen bei einer Temperatur von 20°C bis 250°C in Gegenwart eines sauren Katalysators und eines polaren Lösungsmittels umgesetzt werden, dadurch gekennzeichnet, dass das polare Lösungsmittel Schwefeldioxid ist.

2. Ein Verfahren wie in Anspruch 1 beansprucht, dadurch gekennzeichnet, dass der Reaktionsdruck nicht höher als 20 MPa ist.

3. Ein Verfahren wie in Anspruch 1 oder Anspruch 2 beansprucht, dadurch gekennzeichnet, dass die Reaktionstemperatur im Bereich von 100°C bis 160°C liegt.

4. Ein Verfahren wie in irgendeinem der Ansprüche 1 bis 3 beansprucht, dadurch gekennzeichnet, dass das polare Lösungsmittel ein Gemisch von Schwefeldiodid und einem polaren organischen Co-Lösungsmittel ist.

5. Ein Verfahren wie in irgendeinem der Ansprüche 1 bis 4 beansprucht, dadurch gekennzeichnet, dass das Olefin ein $C_6$—$C_{20}$-Olefin ist.

6. Ein Verfahren wie in irgendeinem der Ansprüche 1 bis 5 beansprucht, dadurch gekennzeichnet, dass das molare Verhältnis von Olefin:(Alkandiol oder Oxa-alkandiol):saurem Katalysator (Wasserstoff-

äquivalent):Schwefeldioxid 1.1 bius 30:0,01 bis 10:1 bis 100 ist.

7. Ein Verfahren wie in irgendeinem der Ansprüche 1 bis 6 beansprucht, dadurch gekennzeichnet, dass der saure Katalysator eine Bronsted-Säure mit einem pKa-Wert von nicht mehr als 2,5 ist.

8. Ein Verfahren wie in irgendeinem der Ansprüche 1 bis 6 beansprucht, dadurch gekennzeichnet, dass der saure Katalysator eine Lewis-Säure ist, die eine Bildungswärme mit Pyridin exothermischer als 9 Kcal/Mol hat.